Europäisches Patentamt

**European Patent Office**

**Office européen des brevets**

(11) Numéro de publication : **0 019 554**
**B1**

(12)

# FASCICULE DE BREVET EUROPÉEN

(45) Date de publication du fascicule du brevet :
**05.10.83**

(51) Int. Cl.³ : **B 01 D 35/00, A 61 M   5/14**

(21) Numéro de dépôt : **80400684.9**

(22) Date de dépôt : **16.05.80**

(54) **Dispositif de filtration.**

(30) Priorité : **17.05.79 FR 7912619**

(43) Date de publication de la demande :
**26.11.80 Bulletin 80/24** '

(45) Mention de la délivrance du brevet :
**05.10.83 Bulletin 83/40**

(84) Etats contractants désignés :
**DE GB**

(56) Documents cités :
**DE C 525 758**
**FR A 2 004 754**
**FR A 2 285 920**
**FR A 2 305 196**
**US A 2 776 055**
**US A 3 465 784**

(73) Titulaire : **Le Boeuf, Lola**
**9 rue Aristide Briand**
**F-95240 Cormeilles en Parisis (FR)**

(72) Inventeur : **Le Boeuf, Guy**
**9 rue Aristide Briand**
**F-95240 Cormeilles en Parisis (FR)**

(74) Mandataire : **Thibon-Littaye, Annick**
**11 rue de l'Etang**
**F-78160 Marly le Roi (FR)**

Jouve, 18, rue St-Denis, 75001 Paris, France

## Dispositif de filtration

La présente invention concerne les dispositifs de filtration d'un liquide physiologique en médecine. Elle a pour objet un dispositif de filtration dont la conception vise en particulier à le rendre mieux adapté que les dispositifs déjà connus à la filtration du sang dans les installations de transfusions, notamment celles des salles d'opération.

Les filtres utilisés dans ce genre d'installation doivent être faciles à installer rapidement sur le circuit de sang alimentant la seringue d'injection, et être peu coûteux pour pouvoir être jetés après usage. Il convient en particulier, d'éviter qu'apparaisse un colmatage du filtre qui obstruerait rapidement la zone du passage du sang et réduirait le débit. Il est de plus usuel dans les installations de transfusion, de disposer sur le circuit de sang, un filtre destiné à retenir les matières solides et un piège à air retenant les bulles d'air.

De tels dispositifs de filtration sont connus et décrits dans la demande de brevet FR-A-2 285 920 déposée au nom de Johnson & Johnson et Purolator Inc. Ce dispositif comporte une soupape unidirectionnelle obturant un trou, et on doit appuyer sur la soupape pour permettre, à l'air contenu dans le filtre de s'échapper par le trou et au sang de remplir le filtre. Quand on a atteint le niveau souhaité, on relâche la soupape qui se ferme automatiquement.

L'invention a pour but de pallier entre autres les inconvénients de l'art antérieur, en réalisant un dispositif de filtration ne nécessitant pas une intervention de l'opérateur pour évacuer l'air contenu dans le filtre et éviter que l'air soit entraîné avec le sang et injecté aux malades en cours de traitement.

Le dispositif selon l'invention convenant à la filtration d'un liquide physiologique, tel que du sang dans les installations de transfusions comporte une chambre de circulation du liquide divisée en au moins deux compartiments par un ensemble filtrant interposé sur le trajet du liquide, et, comporte en outre, dans une zone d'accumulation de l'air présent ou séparé du liquide, une valve montée sur un passage ménagé à travers la paroi de la chambre de filtration. Cette valve comportant un obturateur solidaire d'un flotteur propre à être entraîné par la surface libre du liquide, ledit obturateur coopérant avec deux sièges de valve opposés pour fermer le passage, respectivement en l'absence de surpression gazeuse à l'intérieur de la chambre et sous la poussée du liquide entraînant le flotteur lorsque le niveau libre arrive à proximité de la valve, la valve assurant ainsi automatiquement une évacuation sélective unidirectionnelle de l'air en cas de surpression. La chambre forme un récipient allongé d'écoulement vertical du liquide à filtrer, notamment du sang, entre un raccord d'entrée débouchant au sommet du récipient et un raccord de sortie débouchant au fond du récipient. Ces raccords se disposent de part et d'autre latéralement par rapport à un ensemble filtrant qui comprend au moins un tamis divisant verticalement le récipient sur toute sa hauteur.

La chambre forme un récipient allongé d'écoulement vertical du liquide à filtrer, notamment du sang, entre un raccord d'entrée débouchant au sommet du récipient et un raccord de sortie débouchant au fond du récipient. Ces raccords se disposent de part et d'autre latéralement par rapport à un ensemble filtrant qui comprend au moins un tamis divisant verticalement le récipient sur toute sa hauteur.

La valve du dispositif selon l'invention est alors avantageusement disposée au sommet du récipient, dans le compartiment de sortie, c'est-à-dire de l'autre côté de l'ensemble filtrant par rapport au raccord d'entrée du liquide. C'est là que s'accumule l'air, à l'opposé du ou des bassins de sédimentation sélective formés par la partie inférieure du récipient, suivant le nombre de tamis que peut comporter l'ensemble filtrant, tandis que le récipient se remplit progressivement en cours de fonctionnement, le niveau libre de liquide s'élevant au fur et à mesure que le colmatage de l'ensemble filtrant progresse du bas vers le haut.

Selon un mode de réalisation préféré du dispositif de filtration selon l'invention, l'ensemble filtrant comprend une série de plusieurs tamis, deux à huit par exemple, tous disposés verticalement à travers le récipient, mais distants les uns des autres, et présentant des diamètres de vides (trous du tamis) progressivement variables de l'un à l'autre. Grâce à cette disposition, on assure une organisation encore meilleure de la circulation du fluide, et surtout, par un effet de sédimentations sélectives, on assure une répartition des particules qui colmatent les tamis, en fonction de leurs dimensions, qui permet de prolonger considérablement la durée d'utilisation possible sans avoir besoin d'allonger exagérément la hauteur du dispositif.

Dans sa réalisation pratique, le récipient du dispositif selon l'invention peut être construit sous des formes très variées. La forme de réalisation particulière la plus simple utilise des tamis plans dans un récipient cylindrique ou parallélépipédique. Mais il peut aussi être avantageux de prévoir des tamis cylindriques en liaison avec soit l'entrée soit la sortie de fluide dans l'axe du dispositif et l'autre près du pourtour. Naturellement, il peut être prévu plusieurs entrées et/ou plusieurs sorties, dans les mêmes dispositions relatives par rapport à l'ensemble filtrant. Il reste toujours souhaitable que chaque entrée débouche verticalement, mais latéralement par rapport aux tamis ou autres filtres, de sorte que le sang ne puisse pas tomber directement sur ces derniers.

Dans toutes les variantes du dispositif de filtration selon l'invention, concernant la disposition particulière de l'ensemble filtrant, celle de la valve et leur disposition relative, cette valve remplit essentiellement trois fonctions. En se référant au cas de la filtration du sang, elle laisse sortir l'air du récipient vers l'extérieur quand le sang circule à travers le filtre puisqu'une surpression d'air dans la zone d'accumulation provoque l'ouverture de la valve, mais elle empêche aussi l'air atmosphérique, non stérile, de rentrer dans le récipient, quand celui-ci se trouve en dépression par rapport à l'extérieur, ce qui peut se produire par exemple lorsque la circulation du sang est assurée au moyen d'une pompe péristaltique disposée en aval du filtre. La troisième fonction de la valve est d'empêcher le sang de sortir du dispositif lorsque le récipient arrive à être plein de sang. L'invention permet ainsi d'éliminer toute trace d'air du sang beaucoup mieux que ne le ferait un piège à air disposé en aval du filtre, d'éviter que l'air séparé dans le filtre s'emprisonne en haut de celui-ci et s'y comprime suffisamment pour se mélanger au sang et être entraîné avec lui, et d'éviter qu'il se forme une poche d'air entraînant une diminution de la capacité du récipient et du pouvoir de filtration du dispositif.

Selon d'autres caractéristiques de l'invention, les trois fonctions précisées ci-dessus sont avantageusement assurées au moyen d'une valve comportant un obturateur coopérant avec deux sièges de valve opposés pour fermer le passage, respectivement en l'absence de surpression gazeuse à l'intérieur de la chambre et sous la poussée du liquide entraînant le flotteur lorsque le niveau libre arrive à proximité de la valve, la valve assurant ainsi automatiquement une évacuation sélective unidirectionnelle de l'air en cas de surpression.

D'autres caractéristiques de l'invention, concernant aussi bien la valve elle-même que d'autres particularités de construction du dispositif de filtration sur lequel elle est montée. Il est fait référence, dans cette description, aux figures 1 à 6 des dessins annexés dans lesquels :

La Figure 1 représente une vue générale, partiellement éclatée, d'un filtre à sang réalisé conformément à l'invention ;

La Figure 2 représente le filtre de la Figure 1 en vue latérale ;

La Figure 3 représente le même filtre vu en coupe transversale selon III-III de la Figure 2 ;

La Figure 4 montre le détail de la réalisation de la valve, dans une première forme de réalisation ;

La Figure 5 montre, en coupe partielle, une seconde forme de réalisation de la valve ; et

La Figure 6 représente une troisième forme de réalisation d'une valve utilisable sur le filtre de la Figure 1.

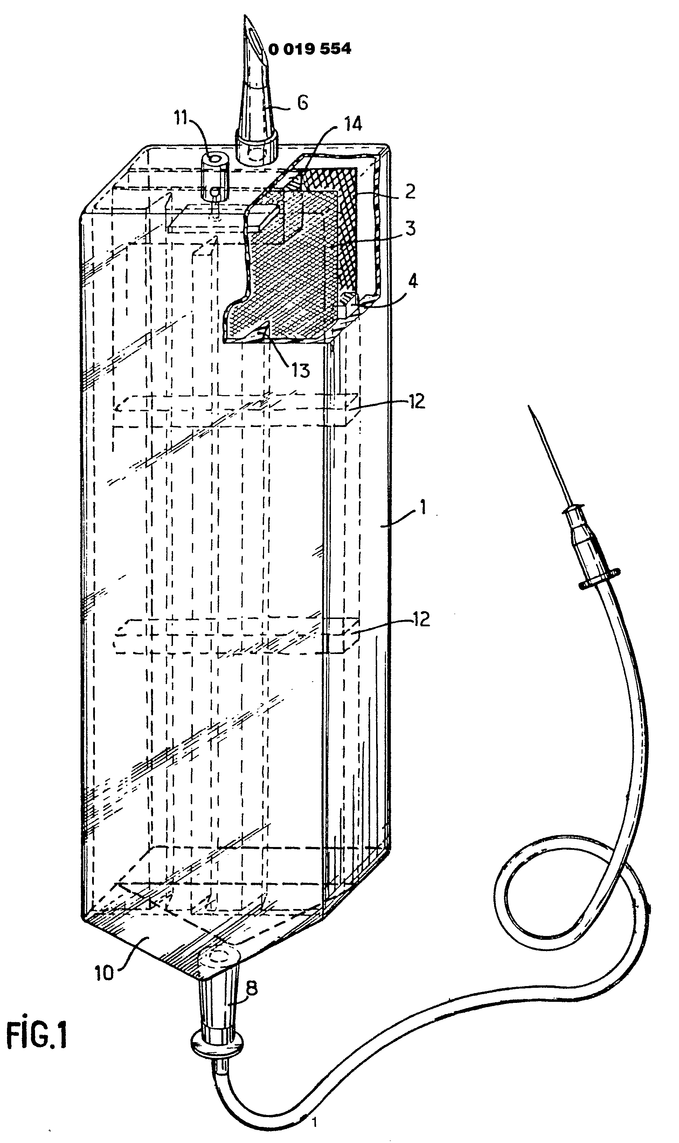

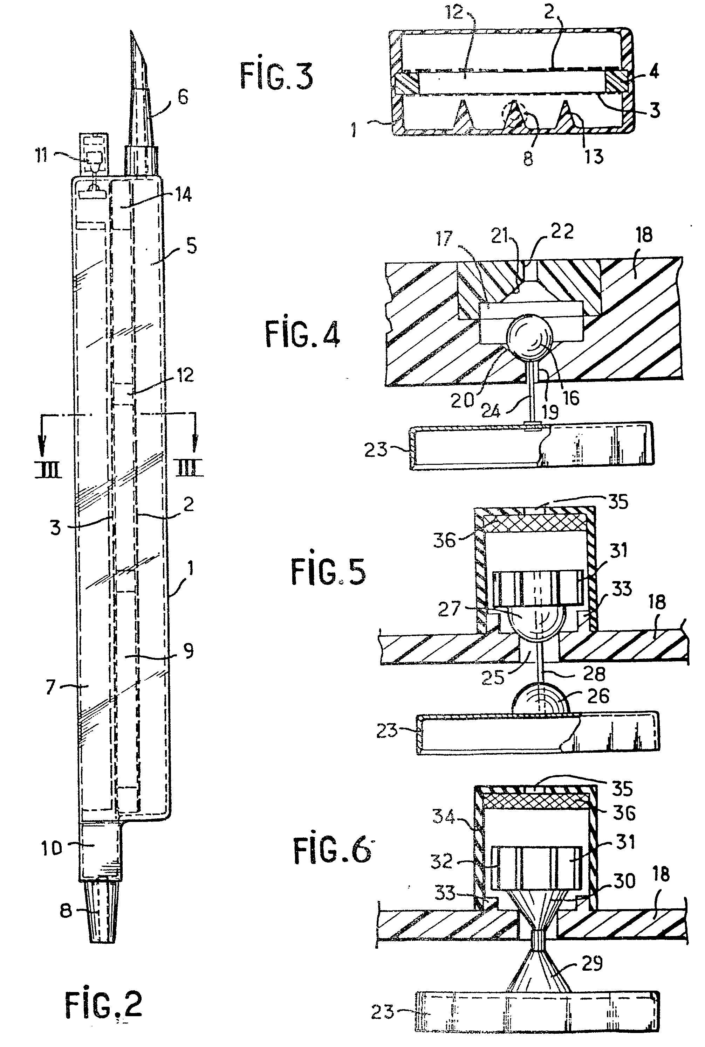

Le filtre décrit, plus spécialement destiné à être utilisé pour le traitement du sang humain dans les installations de transfusion, comprend un récipient 1 qui délimite une chambre parallélépipédique allongée, représentée sur les Figures 1 et 2 telle qu'elle se présente dans sa position d'utilisation, c'est-à-dire orientée verticalement dans le sens de sa longueur. Ce récipient est divisé entièrement dans le sens de sa longueur par un ensemble filtrant qui comporte, dans le cas particulier représenté, deux tamis filtrants 2 et 3. Ces tamis sont montés au moyen d'un cadre rigide 4, engagé dans des rainures des faces latérales du récipient 1. Ils se disposent parallèlement les uns aux autres, dans des plans verticaux.

Les tamis 2 et 3 divisent longitudinalement le récipient 1 en un compartiment d'entrée du sang 5, dans lequel débouche verticalement un raccord d'alimentation 6 à travers la face supérieure du dispositif, un compartiment de sortie du sang 7, dans lequel débouche un raccord de sortie 8 traversant verticalement le fond du récipient, et un compartiment intermédiaire 9, entre les deux tamis. Une valve 11, à effets multiples, est montée sur le dispositif au sommet du compartiment de sortie 7, sur une ouverture ménagée verticalement à travers la face supérieure du récipient 1. Les deux tamis 2 et 3 sont fixés (par collage ou surmoulage) sur les faces extérieures du cadre 4 qui les porte. Les trous du tamis 2, disposé du côté du compartiment d'entrée 5, sont plus gros que ceux du tamis 3, plus fin, qui se trouve du côté du compartiment de sortie 7. Le cadre 4 comporte des entretoises 12 qui maintiennent l'écartement entre les deux tamis, et de plus, pour éviter une déterioration du tamis fin 3 sous l'effet d'une trop forte poussée du liquide passant à travers les tamis, la paroi du compartiment de sortie 7 est pourvue intérieurement d'ailettes longitudinales 13. En bas du filtre, la face inférieure du récipient 1 forme un fond plat aux compartiments 5 et 9, tandis que sous le seul compartiment de sortie 7, elle se prolonge en pointe vers le bas, jusqu'au raccord de sortie 8, en constituant ainsi un fond collecteur de sang 10.

En fonctionnement, la conduite de sortie est raccordée à une seringue d'injection comme schématiquement illustré sur la Figure 1, et le sang est admis par gravité ou par pompage par la conduite d'entrée à un débit réglé régulièrement pour équilibrer le débit soutiré. Le récipient 1 fait en même temps office de pièges à bulles, permettant la séparation de l'air et du sang dans la partie supérieure de l'appareil, et de bassin de sédimentation, par le fait qu'on laisse se former un niveau libre de liquide. Les impuretés entraînées avec le sang sont retenues avec effet sélectif, par les tamis qui représentés au nombre de deux, peuvent être plus nombreux, par exemple au nombre de cinq, laissant passer sélectivement les particules de 400 microns, 200 microns, 100 microns, 50 microns et 25 microns respectivement et séparés les uns des autres d'une distance de l'ordre de 4 mm. D'une part, la zone locale des tamis active dans la filtration se situe préférentiellement au fond de la partie du récipient jouant le rôle de bassin de sédimentation. Le colmatage commence par le fond et il ne progresse que lentement vers le haut, la zone active se déplaçant progressivement au fur et à mesure que progresse le colmatage. D'autre part, les impuretés sont sélectivement retenues par l'un ou l'autre des filtres suivant leurs dimensions, les intervalles entre les tamis adjacents constituant des bacs de sédimentation sélectifs pour les particules de dimensions progressivement décroissantes. Dans la gamme des dimensions de mailles, un tamis conserve donc une efficacité optimale même si un tamis plus grossier est déjà colmaté dans la même zone, et vice-versa. Le niveau libre se règle automatiquement à des hauteurs qui peuvent diminuer d'un bac à l'autre. La sélectivité ainsi assurée permet d'obtenir finalement une efficacité de filtration optimale pour une perte de charge restant très faible et ne variant guère pendant toute la période d'utilisation du dispositif.

A propos de la construction du filtre des Figures 1 et 2 dans sa partie supérieure, on remarquera que dans la zone médiane les ailettes 13 sont arrêtées avant le sommet du récipient 1, pour ne pas gêner le fonctionnement de la valve 11, tandis que le côté supérieur 14 du cadre 4 présente une hauteur suffisante pour empêcher une communication trop directe à travers les tamis, à ce niveau où, en fonctionnement, le sang pénètre dans le dispositif dans le compartiment d'entrée 5, tandis que l'air éventuellement entraîné se réunit en partie haute du compartiment de sortie 7.

La valve 11 sera maintenant décrite dans trois variantes de réalisation, représentées sur les figures 4, 5 et 6 respectivement. Dans toutes ces variantes, cette valve comporte toujours un obturateur à double effet, coopérant avec deux sièges opposés de la paroi limitant la chambre de filtration, pour fermer le passage ménagé à travers cette paroi entre la chambre et l'atmosphère extérieure lorsque cet obturateur, mobile verticalement, occupe respectivement chacune de deux positions extrêmes, alors que pour les positions intermédiaires ce passage est ouvert. De plus, cet obturateur est dans tous les cas solidaire d'un flotteur propre à être soulevé par le liquide lorsque la surface libre de celui-ci arrive à son niveau.

Conformément à la Figure 4, l'obturateur est constitué par une bille sphérique 16 qui est enfermée dans un logement 17 ménagé à l'intérieur de la paroi 18 constituant la face supérieure du récipient 1. Autour d'un conduit 19 par lequel il communique avec l'intérieur du compartiment de sortie 7 du récipient 1, le logement 17 est limité par une surface conique qui constitue un premier siège 20 pour la bille 16. Un deuxième siège 21, de conicité opposée, est formé autour du conduit 22 qui fait communiquer le logement 17 avec l'extérieur. Un flotteur rectangulaire creux 23 est suspendu à la bille 16 par une tige 24 qui passe dans l'axe du conduit 19 sans obturer ce dernier.

On comprend qu'en fonctionnement, l'obturateur constitué par la bille 16 se trouve normalement appuyé contre le siège inférieur 20 sous l'effet de son poids propre additionné de celui du flotteur 23, tandis que lorsque la surpression d'air au sommet du compartiment de sortie 7 devient suffisante pour compenser ce poids, la bille se soulève de son siège et laisse ainsi échapper l'air par les conduits 19 et 22. Mais à l'inverse, en cas de dépression dans le filtre, la bille ne peut qu'être appliquée plus fermement sur le siège 20.

Par contre, lorsque le niveau libre du sang remplissant le filtre s'élève suffisamment pour entraîner avec lui le flotteur 23, la bille se soulève avec le flotteur, mais vient alors fermer la communication avec l'extérieur en s'appuyant sur le siège supérieur 21, empêchant ainsi le passage du sang.

Dans le cas de la figure 5, les deux sièges de l'obturateur sont formés par les bords mêmes du passage 25 ménagé à travers la paroi 18, de chaque côté de cette paroi, et l'obturateur est constitué en deux parties disposées respectivement de part et d'autre de cette paroi et reliées ensemble à travers le passage 25. Sur la figure, les deux parties d'obturateur sont en forme de demi-sphères 26 et 27 et reliées par une tige 28. Le flotteur 23, analogue à celui de la Figure 4, est soudé sur la demi-sphère 26. Les rôles des sièges sont intervertis par rapport à ceux de la Figure 4 : en l'absence d'une surpression gazeuse, la demi-sphère 27 ferme la valve au niveau du siège supérieur, tandis que c'est la demi-sphère inférieure 26 qui vient s'appuyer sur le siège inférieur sous l'effet d'une poussée du liquide, s'exerçant par l'intermédiaire du flotteur 23.

Le même mode de fonctionnement se retrouve dans la Figure 6 qui ne diffère de la Figure 5 que par la forme de l'obturateur, les deux demi-sphères 26 et 27 étant remplacées par des cônes 29 et 30 opposés par leur sommet. En outre, aussi bien dans la variante de la Figure 5 que dans celle de la Figure 6, la partie supérieure de l'obturateur (27 ou 30) est surmontée d'une tête 31, cylindrique et munie de nervures verticales 32. Pendant le fonctionnement normal de la valve, la tête 31 se déplace verticalement avec l'obturateur, au-dessus d'une protubérance annulaire 33 de la paroi 18. Autour de cette même protubérance se monte un guide cylindrique 34, en matière souple, qui surmonte la tête de valve 31 sans gêner son fonctionnement normal. Le guide 34 est fermé à son extrémité, à l'exception d'un orifice 35 qui permet le passage de l'air lorsque la valve est ouverte. Un filtre bactériologique 36, monté à l'intérieur du guide 34, recouvre cet orifice.

Le guide 34 est suffisamment souple pour que l'utilisateur puisse, en le déformant, atteindre manuellement la tête 31 de la valve. Cette possibilité s'utilise en fin de fonctionnement du filtre, après une transfusion, pour vider complètement le filtre du sang qui y reste présent, en laissant pénétrer l'air atmosphérique par la valve. Pour décoller l'obturateur de son siège, il suffit que l'opérateur pousse d'un doigt, latéralement, la tête 31 qui dépasse de la paroi 18 à l'intérieur du guide souple 34. Eventuellement, l'obturateur peut en outre basculer lorsque la tête 31 vient à prendre appui d'un côté sur la protubérance annulaire 33.

**Revendications**

1. Dispositif de filtration d'un liquide physiologique, tel que du sang dans les installations de transfusions, comportant une chambre de circulation du liquide divisée en au moins deux compartiments (5, 7) par un ensemble filtrant (2, 3) interposé sur le trajet du liquide, et, en outre, dans une zone d'accumulation de l'air présent ou séparé du liquide, une valve (11) montée sur un passage (25) ménagé à travers la paroi de la chambre de filtration, caractérisé en ce que ladite valve comporte un obturateur (16 ; 26, 27 ; 29, 30) solidaire d'un flotteur (23) propre à être entraîné par la surface libre du liquide, ledit obturateur coopérant avec deux sièges (20, 21) de valve opposés pour fermer ledit passage (25), respectivement en l'absence de surpression gazeuse à l'intérieur de ladite chambre et sous la poussée dudit liquide entraînant le flotteur lorsque le niveau libre arrive à proximité de la valve, ladite valve assurant ainsi automatiquement une évacuation sélective unidirectionnelle de l'air en cas de surpression.

2. Dispositif selon la revendication 1, caractérisé en ce que ledit obturateur (26, 27 ; 29, 30) est solidaire d'une tête (31) extérieure à la chambre pour l'ouverture manuelle de la valve (11).

3. Dispositif selon la revendication 2, caractérisé en ce que la valve (11) est protégée à l'extérieur de la chambre par un guide élastique (34) recouvrant ladite tête (31).

4. Dispositif selon la revendication 3, caractérisé en ce que le guide élastique (34) contient un filtre bactériologique (36) protégeant une ouverture de passage de l'air (35).

5. Dispositif selon l'une quelconque des revendications 1 à 4, caractérisé en ce que l'obturateur est constitué en deux parties (29, 30) solidaires l'une de l'autre se disposant de part et d'autre de la paroi de la chambre, lesdits sièges opposés étant formés respectivement de chaque côté de la paroi (18) par les bords dudit passage (25).

6. Dispositif selon l'une quelconque des revendications 1 à 5, caractérisé en ce que ladite chambre forme un récipient (1) allongé d'écoulement vertical du liquide, dans lequel ledit ensemble filtrant comprend au moins un tamis vertical (2), et en ce que ladite valve (11) est disposée au sommet du compartiment de sortie (7) du liquide.

7. Dispositif de filtration selon la revendication 6, caractérisé en ce qu'il comprend un raccord d'alimentation (6) débouchant verticalement au sommet du récipient (1) et un raccord de sortie (8) débouchant verticalement au fond du récipient, lesdits raccords (6, 8) se disposant de part et d'autre latéralement par rapport audit ensemble filtrant et en ce que ce dernier comprend au moins deux tamis (2, 3) divisant le récipient (1) verticalement du sommet jusqu'au fond.

8. Dispositif selon la revendication 6 ou 7, caractérisé en ce que l'ensemble filtrant comprend plusieurs tamis (2, 3), parallèles et distants les uns des autres, présentant des dimensions de vide différentes.

9. Dispositif selon la revendication 7 ou 8, caractérisé en ce que chaque tamis (2, 3) est supporté par un cadre rigide (4) comportant des entretoises (12) propres à maintenir écartés des tamis adjacents.

10. Dispositif selon la revendication 6, 7, 8 ou 9, caractérisé en ce qu'il comporte des moyens (14) pour empêcher un écoulement direct du liquide à travers l'ensemble filtrant (2, 3) au niveau de ladite zone d'accumulation d'air.

11. Dispositif selon l'une des revendications 7 à 10, caractérisé en ce que le compartiment de sortie (7) du liquide comporte un fond collecteur de liquide (10).

**Claims**

1. A device for filtering a physiological liquid, such as blood in blood transfusion installations, comprising a chamber for the circulation of said liquid, a filtering unit (2, 3) mounted across the liquid path and dividing said chamber into at least two compartments (5, 7), and in an area where the air present or separated from the liquid has accumulated, a valve (11) mounted on a passage way (25) through the filtration chamber wall, said valve being provided with an obturator (16 ; 26, 27 ; 29, 30) integral with a float (23) adapted to be lifted by the free level of said liquid, the said obturator cooperating with two opposed valve-seats (20, 21) for closing said passage way (25), respectively in the absence of any gas overpressure inside said chamber and under the thrust exerted by said liquid driving said float, whenever said free level is in the vicinity of said valve, said valve automatically ensuring the one-way selective exhaust of air should any overpressure take place.

2. A device according to claim 1, wherein said obturator (26, 27 ; 29, 30) is integral with a head (31) mounted outside said chamber for manual operation of said valve (11).

3. A device according to claim 2, wherein said valve (11) is protected outside said chamber by a resilient guide-member (34) covering said head (31).

4. A device according to claim 3, wherein said resilient guide-member (34) contains a bacteriological filter (36) adapted to shield an opening for the passage of air (35).

5. A device according to any claim from 1 to 4, wherein said obturator comprises two mutually integral portions (29, 30), each of which is situated on one side of the wall of said chamber, the said opposed valve-seats being constituted on either side of said wall (18) by the end edges of said passage (25).

6. A device according to any claim from 1 to 5, wherein the said chamber constitutes an elongated container (1) for the vertical flow of said liquid, in which said filtering unit comprises at least one vertical sieve (2), and wherein the said valve (11) is arranged at the top of that compartment through which said liquid is drained from said device.

7. A filtration device according to claim 6, comprising an inlet connecting member (6) protruding vertically from the container top (1) and an outlet connecting member (8) protruding vertically from the bottom of said container, the said connecting members (6, 8) being laterally arranged on both sides of said filtering unit, the said filtering unit comprising at least two sieves (2, 3) adapted to divide said container (1) vertically along the full height thereof.

8. A device according to claim 6 or 7, wherein said filtering unit comprises a plurality of sieves (2, 3) positioned in parallel and spaced relationship to one another, the diameters of the holes of said sieves varying from one sieve to the next one.

9. A device according to claim 7 or 8, wherein each sieve (2, 3) is carried by a rigid frame (4) provided with cross-pieces (12) adapted to maintain adjacent sieves in spaced relationship.

10. A device according to claim 6, 7, 8 or 9, wherein are provided means (14) for preventing the said liquid from flowing directly through said filtering unit (2, 3) at the level of said air accumulation area.

11. A device according to any claim from 7 to 10, wherein the liquid outlet compartment (7) is provided with a liquid collecting bottom (10).

**Ansprüche**

1. Vorrichtung zum Filtern einer physiologischen Flüssigkeit, wie Blut in Transfusionsanlagen, mit einer Flüssigkeitsumlaufkammer, die in mindestens zwei Abteile (5, 7) durch eine Filterbaugruppe (2, 3) unterteilt ist, die in den Flüssigkeitsweg eingeschaltet ist und mit einem in einer mit der Flüssigkeit verbundenen oder von ihr getrennten Luftakkumulationszone angeordneten Ventil (11), das in einem sich durch die Wandung der Filterkammer erstreckenden Durchlaß (25) montiert ist, dadurch gekennzeichnet, daß das Ventil ein Sperrglied (16, 26, 27, 29, 30) umfasst, verbunden mit einem Schwimmer (23), der von dem Flüssigkeitsspiegel mitgenommen wird, wobei das Sperrglied mit zwei Ventilsitzen (20, 21), zusammenwirkt, die einander entgegengesetzt für das Absperren des Durchlasses (25) ausgebildet sind und zwar bei Fehlen von Gasüberdruck im Innern der Kammer, bzw. unter dem Druck der den Schwimmer mitnehmenden Flüssigkeit, sobald der freie Pegel nahe dem Ventil ankommt, und wobei das Ventil auf diese Weise automatisch eine selektive unidirektionelle Evakuation der Luft im Falle von Überdruck sicherstellt.

2. Vorrichtung nach Anspruch 1, dadurch gekennzeichnet, daß das Sperrglied (26, 27, 29, 30) mit einem äußeren Kopf (31) der Kammer für das manuelle Öffnen des Ventils (11) verbunden ist.

3. Vorrichtung nach Anspruch 2, dadurch gekennzeichnet, daß das Ventil (11) nach außen bezüglich der Kammer durch eine elastische Führung (34) geschützt ist, die den Kopf (31) abdeckt.

4. Vorrichtung nach Anspruch 3, dadurch gekennzeichnet, daß die elastische Führung (34) ein bakteriologisches Filter (36) enthält, das eine Luftdurchtrittsöffnung (35) schützt.

5. Vorrichtung nach einem der Ansprüche 1 bis 4, dadurch gekennzeichnet, daß das Sperrglied aus zwei Teilen (29, 30) besteht, die miteinander verbunden sind und sich beidseits der Wandung der Kammer befinden, wobei die einander entgegengesetzten Ventilsitze auf der einen bzw. der anderen Seite der Wandung (18) durch die Ränder des Durchlasses (25) gebildet sind.

6. Vorrichtung nach einem der Ansprüche 1 bis 5, dadurch gekennzeichnet, daß die Kammer einen langgestreckten vertikalen Flüssigkeitsablaufrezipienten (1) bildet, in welchem die Filterbaugruppe mindestens ein vertikales Sieb (2) umfasst, und daß das Ventil (11) an der höchsten Stelle des Auslaßabteils (7) der Flüssigkeit angeordnet ist.

7. Vorrichtung zum Filtern nach Anspruch 6, dadurch gekennzeichnet, daß sie einen Speiseanschluß (6) umfasst, der vertikal an der höchsten Stelle des Rezipienten (1) mündet und einen Auslaßanschluß (8) umfasst, der vertikal am Boden des Rezipienten mündet, und daß die beiden Anschlüsse (6, 8) sich seitlich auf der einen bzw. der anderen Seite bezüglich der Filterbaugruppe befinden, und daß die letztere mindestens zwei Siebe (2, 3) umfasst, die den Rezipienten (1) vertikal von seiner höchsten Stelle bis zum Boden unterteilen.

8. Vorrichtung nach einem der Ansprüche 6 oder 7, dadurch gekennzeichnet, daß die Filterbaugruppe mehrere Siebe (2, 3) umfasst, die parallel im Abstand voneinander angeordnet sind und unterschiedliche Porenabmessungen aufweisen.

9. Vorrichtung nach einem der Ansprüche 7 oder 8, dadurch gekennzeichnet, daß jedes Sieb (2, 3) von einem starren Rahmen (4) abgestützt ist, der Streben (12) umfasst, um benachbarte Siebe im Abstand voneinander zu halten.

10. Vorrichtung nach einem der Ansprüche 6, 7, 8 oder 9, dadurch gekennzeichnet, daß sie Mittel (14) umfasst zum Unterbinden eines direkten Abfließens von Flüssigkeit quer zur Filterbaugruppe (2, 3) in Höhne der Luftsammelzone.

11. Vorrichtung nach einem der Ansprüche von 7 bis 10, daduch gekennzeichnet, daß das Auslaßabteil (7) der Flüssigkeit einen Flüssigkeitssammelboden (10) umfasst.

**FIG.1**

0 019 554

FIG.3

FIG.4

FIG.5

FIG.6

FIG.2

2